# EUROPÄISCHE PATENTSCHRIFT

(11) **EP 0 442 072 B1**
(45) Veröffentlichungstag und Bekanntmachung des Hinweises auf die Patenterteilung: **18.11.1993**
(21) Anmeldenummer: 90123773.5
(22) Anmeldetag: 11.12.1990
(51) Int. Cl.: A61M 39/04

(54) **Ansatzstück für medizinische Leitungen**
Connector for medical conduits
Elément de raccordement pour conduits médicaux

(30) Priorität: 12.01.1990 DE 4000764
(43) Veröffentlichungstag der Anmeldung: 21.08.1991
(73) Patentinhaber: B. Braun Melsungen AG, 34209 Melsungen (DE)
(72) Erfinder: Herlitze, Gerd, Dipl.-Ing., W-3507 Baunatal 7 (DE); Schmidt, Klaus-Joachim, W-3508 Ahnatal (DE); Lesemann, Egon, Dipl.-Ing., W-3508 Melsungen (DE); Maier, Hans-Otto, Dr. Dipl.-Ing., W-3503 Lohfelden (DE); Voges, Karl-Friedrich, Dipl.-Ing., W-3508 Melsungen (DE); Wiegel, Heinz, Dipl.-Ing., W-6445 Alheim-Heinebach (DE)
(74) Vertreter: Selting, Günther, Dipl.-Ing.

(56) Entgegenhaltungen:
- DE-A- 2 830 800
- DE-A- 3 147 609
- US-A- 4 512 766

## Beschreibung

Die Erfindung bezieht sich auf ein Ansatzstück für eine starre oder flexible medizinische Leitung, bestehend aus einem Gehäuse mit einer an beiden Enden offenen Kammer, deren eines offenes Ende mit der Leitung verbunden ist und deren entgegengesetztes offenes Ende einen Anschlußteil aufnimmt und aus einer Ventilvorrichtung mit einem gummielastischen Ventilkörper in Form eines offenen becherförmigen Hohlzylinders mit geschlitztem Bodenteil.

Unter den Begriff "starre oder flexible medizinische Leitungen" fallen im Rahmen der Erfindung Kanülen aus Metall oder Kunststoff und flexible Katheter, die zur Abnahme von Blut oder zur Zuführung von Blut, Infusionslösungen und Medikamenten in das Gefäßsystem eines Patienten eingesetzt werden. Wenn die Spitze der Leitung in das Gefäß eindringt, strömt Blut zurück und tritt bei den meisten bekannten Ansatzstücken aus diesem aus, was wegen der Übertragungsgefahr von ansteckenden Krankheiten durch Blut vermieden werden soll. Auch bei einem Wechsel einer weiterführenden Leitung durch Abziehen des Anschlußteiles von dem Gehäuse des Ansatzstückes ist dieses offen und Blut kann austreten; bei ungünstiger Lage des Patienten dann auch Luft durch die Öffnung in das Venensystem gelangen und eine Luftembolie auslösen.

Um diesem Mangel zu begegnen, wurde ein Ansatzstück mit Ventilvorrichtung geschaffen (DE 28 17 102), bei dem in einer radial umlaufenden Nut in der Wandung der Kammer des Gehäuses eine Scheibe aus elastomerem Material mit einem zentralen Schlitz gehaltert ist. Ferner befindet sich in der Kammer ein Verstellglied in Form eines konischen Hülsenkörpers, der unter dem Andruck eines in eine Einstecköffnung des Gehäuses einsteckbaren Anschlußteils, z.B. des Ansatzes einer Stahlkanüle, vorgeschoben wird und in dieser vorgeschobenen Stellung gegen die Scheibe drückt und sie zur Öffnung des Schlitzes mindestens teilweise durchsetzt. Dabei ist die Zuverlässigkeit des Verschlusses nach Herausziehen der Stahlkanüle in Frage gestellt, weil er ausschließlich auf der Eigenelastizität des Materials der Scheibe beruht, welche - insbesondere, wenn die Vorrichtung als anwendungsfertiger Set geliefert wird, bei dem eine Stahlkanüle während längerer Lagerzeit die Lippen bildenden glatten Ränder des Schlitzes in der vorgespannten Scheibe deformiert - durch Materialermüdung nachläßt. Deshalb kann es passieren, daß beim Herausziehen der Stahlkanüle die verformten Ränder des Schlitzes nicht nur nicht ganz dicht schließen, sondern sogar ein offenes Loch enthalten, durch das Blut zurücksickert. Um dieses Loch möglichst klein zu halten, werden in der Praxis Stahlkanülen größeren Durchmessers auf der Höhe der Elastomerscheibe in dem Ansatzstück mit einer Einschnürung versehen. Diese Einschnürung ist fertigungstechnisch nur unter großem Aufwand herstellbar und außerdem bildet sie im Innern der Stahlkanüle eine Drosselstelle, so daß bei der Punktion eines Gewebes ausgestanzte Gewebestücke in der Drosselstelle steckenbleiben und der Punktionserfolg nicht durch Blutaustritt sichtbar wird. Der Anwender punktiert das Gefäß unnötigerweise mehrmals und belastet damit den Patienten unangemessen.

Ferner ist gemäß dem Oberbegriff des Anspruchs 1 eine Punktions- und Einführungsvorrichtung bekannt, die eine Ventilvorrichtung mit einem gummielastischen becherförmigen Hohlzylinder aufweist, dessen Bodenteil geschlitzt ist (DE 31 47 609). Dabei dient ein in der Kammer des Gehäuses des Ansatzstückes axial verschiebbares Verstellglied in Form einer an beiden Enden offenen Hülse der Vorspannung des Bodenteils des unbeweglichen becherförmigen Hohlzylinders durch radiale Pressung. Vor der Benutzung der Vorrichtung umgreift das innere Hülsenende nur das patientenferne offene Ende des Hohlzylinders, so daß sein entgegengesetzter Bodenteil während der Lagerzeit entspannt ist und keine Deformationen an den Dichtlippen auftreten, die zu Undichtigkeiten führen könnten. In das dem Anwender zugewandte äußere Ende der Hülse ist ein Anschlußteil, z.B. ein Kanülenansatz, eingesetzt, dessen axiales Vorschieben zur Vorbereitung der Kanülenspitze auf den Punktionsvorgang das innere Hülsenende ganz über den Hohlzylinder schiebt und den Bodenteil radial zusammenpreßt. In dieser Position rastet die Hülse in dem Gehäuse ein - mit der Folge, daß die Lippen des Schlitzes des Bodenteiles nun immer aneinandergepreßt sind und keine Möglichkeit der Entspannung mehr gegeben ist. Um nach Herausziehen der Punktionskanüle einen hochflexiblen Katheterschlauch durch die geschlossengehaltenen Lippen des radial vorgespannten Bodenteiles hindurchschieben zu können oder die Lippen unter dem schwachen Andruck eines Fluidstromes zu öffnen, muß der Boden des Hohlzylinders sehr dünn und nachgiebig sein. In diesem Falle besteht die Gefahr, daß der Schlitz sich auch bei dem schwachen Andruck des Blutrückstromes öffnet und das Ventil seine Rückschlagfunktion nicht erfüllt. Je nach Länge der Liegezeit eines Katheterschlauches verformen sich die dünnen Lippen an dem vorgespannten Bodenteil durch Materialermüdung und auch bei dieser Ventilvorrichtung entsteht durch Deformation ein unverschließbares Loch. Bei den beiden bekannten erläuterten Ventilvorrichtungen von Ansatzstücken verteuert die Notwendigkeit des zusätzlichen Verstellgliedes die Herstellung des Ansatzstückes.

Der Erfindung liegt die Aufgabe zugrunde, eine Ventilvorrichtung der eingangs genannten Art so zu verbessern, daß der geschlitzte Bodenteil des gummielastischen Hohlzylinders in Einführrichtung leicht öffenbar ist und in Rückströmrichtung zuverlässig dicht schließt.

Diese Aufgabe wird erfindungsgemäß dadurch gelöst, daß der Hohlzylinder in der Kammer axial beweglich ist, daß eine Feder den Hohlzylinder in Schließstellung mit seinem Öffnungsrand gegen das innere Ende einer Anschlußteil-Einstecköffnung des Gehäuses andrückt, deren Durchmesser größer als der Innenumfang des Öffnungsrandes des Hohlzylinders ist und daß dabei der Bodenteil in eine angepaßte zentrale Lochöffnung eines in dem Gehäuse fixierten Schließringes hineingezogen ist, aus der der Bodenteil zur Freigabe seiner durch Schlitzung gebildeten Schenkel gegen die Federkraft axial herausdrückbar ist.

Der federbelastete axial bewegliche becherförmige Hohlzylinder der Ventilvorrichtung wird von einem gegen seinen oberen Rand andrückenden Anschlußteil, z.B. einem Ansatz einer Stahlkanüle oder einer Infusionsleitung, direkt betätigt, so daß zusätzliche Verstellglieder fortfallen. Durch die bei Einstecken eines Anschlußteiles in die Einstecköffnung automatisch erfolgende axiale Verschiebung des Hohlzylinders gegen die Kraft der Feder wird sein Bodenteil aus der zentralen Lochöffnung des Schließringes herausgedrückt, so daß die Schenkel des Bodenteiles sich frei und spannungslos nach außen spreizen können, um die Durchlaßöffnung freizugeben. Da der Schließring in Öffnungsposition des Hohlzylinders inaktiv ist und der Bodenteil dann in radialer Richtung unbelastet ist, genügt geringer Öffnungsdruck und das Ansatzstück ist deshalb mit voll wirksamem Rückschlagventil sowohl zum Durchlaß von Kanülen oder Kathetern als auch von Flüssigkeiten geeignet. Wenn der Anschlußteil aus der Einstecköffnung herausgezogen und damit sein axialer Andruck gegen den Hohlzylinder aufgehoben werden, verstellt die Feder den Hohlzylinder sofort in Richtung der Einstecköffnung des Gehäuses, wobei der Bodenteil in die zentrale Lochöffnung des Schließringes hineingezogen wird. Durch die gegenseitige Anpassung von Lochöffnung und Außenumfang des Bodenteiles erfolgt eine Umgürtung des Bodenteiles derart, daß die Ränder der Schlitzung des Bodenteiles spannungsfrei gegeneinander gedrückt gehalten werden, so daß weder von außen Luft eingeschleppt werden noch von innen Blut heraussickern kann. Die Vorspannungsfreiheit des Hohlzylinders sowohl in Öffnungs- als auch in Schließstellung garantiert die einwandfreie Funktion der Ventilvorrichtung bei wiederholter Öffnung und Schließung, weil das Material ohne Ermüdungserscheinungen die ihm innewohnende Elastizität beibehält.

Wenn das erfindungsgemäße Ansatzstück mit einer Venenverweilkanüle ausgestattet und als benutzungsbereites Punktionsbesteck mit einer eingesetzten Stahlkanüle geliefert wird, drückt der in der Anschlußteil-Einstecköffnung steckende Stahlkanülenansatz axial gegen den Hohlzylinder, so daß der Bodenteil über den Schließring vorsteht und seine Schenkel sich unter dem Andruck der Stahlkanüle radial frei nach außen spreizen können, um den Durchgang in dem Hohlzylinder zu öffnen. Dabei ergeben sich keine inneren Materialspannungen und durch die Belastungslosigkeit des Bodenteiles während der Lagerzeit des Punktionsbestecks verformen sich die lose gegen die Stahlkanüle anliegenden Ränder der Schenkel nicht; ihre Schließwirkung nach Herausziehen der Stahlkanüle ist in beiden Richtungen optimal.

In vorteilhafter Ausgestaltung der Erfindung ist vorgesehen, daß der Hohlzylinder mit kreisförmigem Querschnitt von einer Schraubenfeder umgeben ist, deren eines Ende sich auf dem Schließring abstützt und deren anderes Ende an dem Hohlzylinder festgelegt ist. Die Schraubenfeder besteht aus Metall passender Stärke.

Vorteilhafterweise ist der Bodenteil dick ausgebildet und die Schlitzung des Bodenteiles erstreckt sich bis in die Mantelfläche des Hohlzylinders hinein. Auf diese Weise entsteht bei nur einem Schlitz eine Art Schnabelventil mit zwei langen spreizbaren Schenkeln. Da erfindungsgemäß das freie Ende des Bodenteiles einen äußeren Ringkonus aufweist, der in Schließstellung des Hohlzylinders in eine konische Vertiefung an dem patientenseitigen Ende der Lochöffnung des Schließringes im wesentlichen passend eingreift, werden die beiden Schenkel in Abdichtstellung radial gegeneinandergedrückt gehalten und ihre leichtgängige Spreizbarkeit bei Öffnung wirkt sich in Schließstellung nicht nachteilig aus. Die Zuhaltung der Schlitzung sorgt dafür, daß kein Blut nach außen sickern und keine Luft nach innen strömen kann.

In weiterer zweckmäßiger Ausgestaltung der Erfindung ist vorgesehen, daß ein kreiszylindrischer Längskanal in dem Hohlzylinder an der inneren Grundfläche des Bodenteiles in einer symmetrischen Kegelvertiefung endet und an seinem entgegengesetzten offenen Ende einen inneren Ringbund aufweist. Die Kegelvertiefung sorgt für eine Konzentrierung und damit Verstärkung der Öffnungskraft an der Schlitzung in Öffnungsposition des Hohlzylinders. Da die durch Schlitzung des Bodenteiles entstehenden Schenkel gerade Ränder haben, ist ein formgetreues Umschließen eines hindurchgeführten Gegenstandes mit kreisförmigem Querschnitt nicht möglich und es entstehen je nach Art der Schlitzung mindestens zwei offene dreieckige Durchlässe am Umfang des langgestreckten Gegenstandes, durch die Blut hindurchsickern kann. Diese Sickerströme werden durch den inneren Ringbund am patientenfernen Ende des Hohlzylinders zurückgehalten. Das Ansatzstück erfüllt hohe hygienische Sicherheitsanforderungen für den Patienten und für den Anwender.

Die Kammer in dem Gehäuse ist vorzugsweise kreiszylindrisch und der Schließring ist als separates Teil aus zwei halbkreisförmigen Hälften gebildet. Die Teilung des Schließringes dient der Ermöglichung der Montage des Hohlzylinders in dem Ansatzstück. Die zentrale Lochöffnung in dem Schließring ist bevorzugt kreisförmig.

In der Zeichnung sind Ausführungsbeispiele der Erfindung schematisch dargestellt. Es zeigt:
Fig. 1 einen Längsschnitt durch ein Ansatzstück eines Katheters mit geschlossener Ventilvorrichtung,
Fig. 2 einen Querschnitt längs der Linie II-II in Figur 1,
Fig. 3 eine Seitenansicht des Schließringes,
Fig. 4 einen Längsschnitt durch das Ansatzstück einer Venenverweilkanüle, in Verbindung mit einer Punktionsnadel und
Fig. 5 einen Längsschnitt durch das Ansatzstück nach Figur 4 in Verbindung mit einer Infusionsleitung.

Ein Ansatzstück 10 besteht aus einem röhrenförmigen zylindrischen Gehäuse 20 aus Kunststoff mit kreisförmigem innerem und äußerem Querschnitt. Im Inneren des Gehäuses 20 befindet sich eine kreiszylindrische Kammer 11, die an ihrem patientenfernen Ende über eine Ringschulter 12 an eine konische Anschlußteil-Einstecköffnung 13 anschließt, deren Durchmesser kleiner als der Durchmesser der Kammer 11 ist. Das andere Ende der Kammer 11 geht über eine Ringschulter 15 in einen kreiszylindrischen Raum 14 über, dessen Durchmesser größer als der Durchmesser der Kammer 11 ist. Der patientenferne Rand des Gehäuses 20 ist mit zwei diametral gegenüberliegenden, nach außen gerichteten radialen Nasen 16, 17 versehen, die der Verriegelungskupplung mit einem Anschlußteil dienen. Vorzugsweise sind die schrägen Unterflanken der Nasen 16, 17 in Umfangsrichtung gegensinnig abgeschrägt. Auf den patientenseitigen Rand 18 des Gehäuses 20 ist ein Hohlstutzenteil 19 mit einem kappenartigen Ansatz 19a aufgesteckt und mit diesem verschweißt oder verklebt. Mit dem Hohlstutzenteil 19 ist ein flexibler Katheter 21 verbunden, der eine in ein Blutgefäß oder einen Körperhohlraum einführbare medizinische Leitung bildet. Der Katheter 21 ist an seinem hinteren Ende mit einer Ringwulst 22 versehen, die in eine Ringaussparung des Hohlstutzenteiles 19 eingreift und durch Schweißung oder Umspritzung 23 gesichert ist. Ein Adapter 24 mit konischem Durchgang setzt eine Trichteröffnung des Ansatzes 19a fort und leitet z.B. Flüssigkeit in den Katheter 21 hinein.

In der Kammer 11 des Gehäuses 20 befindet sich eine Ventilvorrichtung 30, die bis in den Raum 14 ragt. Sie besteht im wesentlichen aus einem axial verschiebbaren becherförmigen Hohlzylinder 31 mit kreisförmigem Querschnitt aus gummielastischem Material, einer Schraubenfeder 40 aus Metall und einem Schließring 50 aus steifem Werkstoff.

Der Hohlzylinder 31 weist einen kreiszylindrischen Schaft 32 auf, der an einem Ende einen äußeren Profilkranz 33 und am anderen Ende einen axial dicken Bodenteil 34 hat. Der Profilkranz 33 ist auf seinem Außenumfang axial mehrfach abgestuft und sein äußerster Rand 33a liegt abdichtend gegen die Innenfläche der Kammer 11 an. Er befindet sich im Bereich einer Öffnung 35 eines kreiszylindrischen Längskanals 36 des Hohlzylinders 31, der an der inneren Grundfläche des dicken Bodenteiles 34 in einer Kegelvertiefung 37 endet. Der Bodenteil 34 ist an seinem freien Ende mit einem divergierenden äußeren Ringkonus 38 versehen. Seine äußere Bodenfläche 39 ist kreisförmig und eben. Der Längskanal 36 erstreckt sich über die Länge des Schaftes 32 und vor der Öffnung 35 enthält er einen inneren Ringbund 41, der als Dichtung für einen durch den Längskanal 36 hindurchgeführten langgestreckten Gegenstand dient. Der dicke Bodenteil 34 ist durch einen ihn diametral durchschneidenden Längsschlitz 42, der sich bis in den Schaft 32 erstreckt, in zwei Schenkel 43, 44 aufgeteilt, die sich bis zum inneren Ende 45 des Längsschlitzes 42 auseinanderspreizen können. Das innere Ende 45 des Längsschlitzes 42 befindet sich in der Nähe der Quermittellinie des Hohlzylinders 31 in der patientenseitigen Hälfte desselben.

Die Stirnfläche 46 des Profilkranzes 33 ragt radial in den lichten Querschnitt des inneren Endes der Einstecköffnung 13 des Gehäuses 20 hinein und bildet eine Schulter, die nach außen freiliegt, so daß ein in den Innenkonus der Einstecköffnung 13 eingesetzter Anschlußteil mit seinem Rand gegen die Stirnfläche 46 drückt und den Hohlzylinder 31 verschiebt.

Das eine Ende der Schraubenfeder 40 stützt sich an der Metallscheibe 47, die auf der inneren Ringstufe des Profilkranzes 33 aufliegt, ab. Die Schraubenfeder 40 hat über ihre ganze Länge gleichen Durchmesser und ihr anderes Ende drückt gegen den Schließring 50. Aufgabe der Metallscheibe 47 ist es, ein Eindrücken der Schraubenfeder 40 in weicheres Material des Profilkranzes 33 zu verhindern. Die Schraubenfeder 40 drückt den Hohlzylinder 31 mit seiner Stirnfläche 46 gegen die Ringschulter 12 und zieht den Bodenteil 34 in den Schließring 50 hinein.

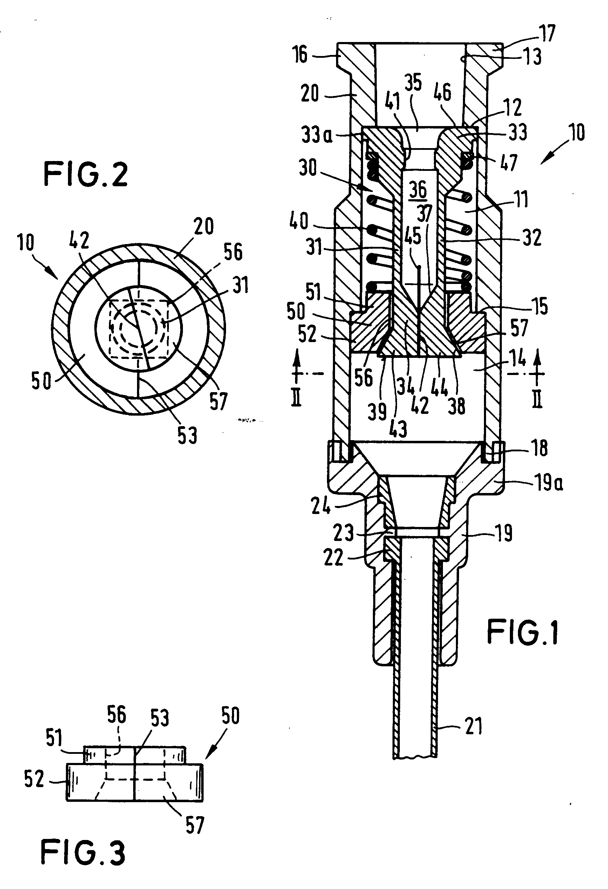

Der Schließring 50 hat zwei kreiszylindrische Abschnitte 51, 52 unterschiedlichen Durchmessers, die koaxial aufeinanderfolgen. Der Abschnitt 51 ist Gegenlager für die Feder 40 und der Abschnitt 52 paßt klemmend und dichtend in den erweiterten Raum 14. Zur Erleichterung der Montage ist der Schließring 50 an einer Teilungslinie 53 diametral geteilt. In der Mitte des Schließringes 50 ist eine kreisförmige oder quadratische Lochöffnung 56 ausgebildet, deren Seiten gegen den kreisförmigen Bodenteil 34 des Hohlzylinders 31 anliegen (Fig. 2). An dem patientenseitigen Ende der Lochöffnung 56 ist eine divergierende konische Vertiefung 57 ausgebildet, in die der äußere Ringkonus 38 des Bodenteiles 34 eingepaßt ist, wenn der Hohlzylinder 31 die Schließstellung gemäß Figur 1 eingenommen hat.

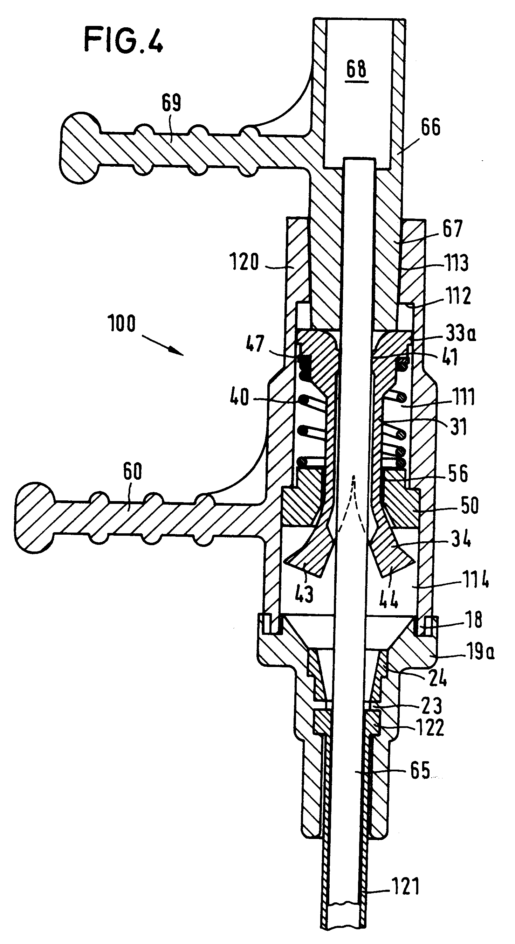

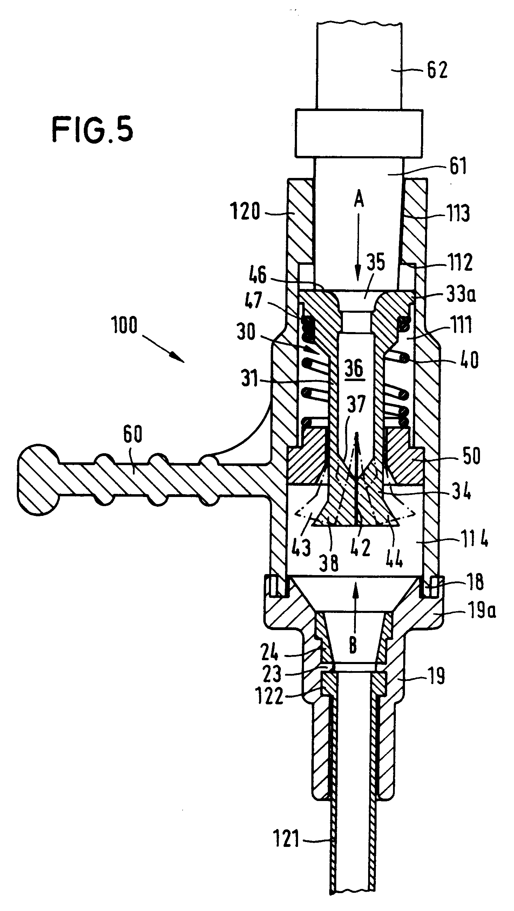

Ein Ansatzstück 100 mit Ventilvorrichtung 30 ist in praktischer Anwendung bei einer Venenverweilkanüle und einer Infusionsleitung in Figuren 4 und 5 gezeigt. Dabei hat das Gehäuse 120, dessen Aufbau dem Gehäuse 20 entspricht, eine radial abstehende Griffplatte 60 und an ihrem patientenseitigen Ende ist eine kurze Venenverweilkanüle 121 mittels einer Ringwulst 122, wie zu Figur 1 beschrieben, befestigt. Die Venenverweilkanüle 121 besteht aus einem dünnwandigen Kunststoffröhrchen, das über eine gewisse Zeit Zugang zu einem Blutgefäß verschafft. Zu diesem Zweck muß zunächst das Blutgefäß punktiert werden. Dies geschieht mittels einer hohlen Punktionsnadel 65 aus Stahl, die an ihrem patientenseitigen Ende eine scharfe angeschliffene Spitze aufweist und an ihrem entgegengesetzten patientenfernen Ende mit einem Ansatz 66 verbunden ist. Der Ansatz 66 weist einen Außenkonus 67 auf, durch den das Ende der Punktionsnadel 65 in einen Hohlraum 68 ragt, der nach außen offen ist und durch einen nicht gezeichneten Blutfängerstopfen zur Anzeige des Punktionserfolges abgedeckt sein kann. Ferner ist der Ansatz 66 mit einer seitwärts gerichteten Griffplatte 69 versehen, die zu der Griffplatte 60 des Gehäuses 120 parallel gerichtet ist.

Das Punktionsbesteck wird wie in Figur 4 gezeigt montiert, steril verpackt und gelagert. In diesem Zustand drückt die Stirnkante des Außenkonus 67 den Hohlzylinder 31 gegen die Kraft der Schraubenfeder 40 von der Ringschulter 112 des Gehäuses 120 weg, so daß die beiden Schenkel 43, 44 von dem Schließring 50 freikommen und sich unter dem Andruck der Punktionsnadel 65 wie gezeigt spreizen. In dieser Position unterliegen die Schenkel 43, 44 keiner Spannung, so daß das Material ermüdungsfrei bleibt und die gegen die Punktionsnadel 65 anliegenden Kanten der geteilten Kegelvertiefung 37 sich nicht deformieren. Wenn mit dem Punktionsbesteck punktiert worden ist, wird die Punktionsnadel 65 zurückgezogen, der Außenkonus 67 gibt den Hohlzylinder 31 frei und er wird von der Schraubenfeder 40 zurückgedrückt, wobei sein Bodenteil 34 in die zentrale Lochöffnung 56 des Schließringes 50 eindringt. Dabei werden die geraden Ränder entlang des Schlitzes 42 gegeneinandergedrückt gehalten und es ergibt sich ein dichter Abschluß in beiden Richtungen.

Wenn die Venenverweilkanüle 121 anschließend mit einer Infusionsleitung gekoppelt werden soll, wird - wie in Figur 5 gezeigt - in die Einstecköffnung 113 ein Außenkonus 61 eines Anschlußteiles eingesteckt, der mit einer Infusionsleitung 62 verbunden ist, die zu einem Infusionsflüssigkeitsbehälter führt. Die Stirnkante des Außenkonus 61 drückt gegen die Stirnfläche 46 des Hohlzylinders 31 und verschiebt ihn gegen die Federkraft axial in Richtung des Raumes 114 des Gehäuses 120. Dabei wird die durch den Rand der Lochöffnung 56 gebildete Bandage zur Zuhaltung des Bodenteiles 34 des Hohlzylinders 31 gewissermaßen abgestreift und die beiden Schenkel 43, 44 können sich radial spreizen (strichpunktiert), wenn ein in Richtung des Pfeiles A strömender Flüssigkeitsstrom gegen die Kegelvertiefung 37 in der inneren Grundfläche des Bodenteiles 34 trifft. Der Flüssigkeitsstrom durchströmt praktisch verwirbelungsfrei die große Öffnung zwischen den beiden Schenkeln 43, 44 und hält diese ausreichend in gegenseitigem Abstand, so daß ein ungehinderter Durchstrom gewährleistet ist. Wenn z.B. beim Wechsel der Infusionsleitung 62 der Außenkonus 61 aus dem Gehäuse 120 herausgezogen wird, drückt die Schraubenfeder 40 unverzüglich den Hohlzylinder 31 nach hinten, bis seine Stirnfläche 46 gegen die Ringschulter 12 anliegt und der äußere Ringkonus 38 des Bodenteiles 34 in die konische Vertiefung 57 des Schließringes 50 hineingezogen ist. Der Schließring 50 sorgt dafür, daß die geraden Ränder des Längsschlitzes 42 über die gesamte Dicke des Bodenteiles 34 zusammengehalten werden (Fig. 1), so daß weder in Richtung des Pfeiles A Luft von außen einströmen noch in Richtung des Pfeiles B Blut zurückströmen kann. Der längsgeschlitzte Bodenteil 34 bildet eine Art Schnabelventil mit durch die Dicke des Bodenteiles 34 bedingten großflächigen Abdichtzonen zwischen den beiden Schenkeln 43, 44.

## Patentansprüche

1. Ansatzstück für eine starre oder flexible medizinische Leitung (21), bestehend aus einem Gehäuse (20) mit einer an beiden Enden offenen Kammer (11), deren eines offenes Ende mit der Leitung (21) verbunden ist und deren entgegengesetztes offenes Ende einen Anschlußteil aufnimmt und aus einer Ventilvorrichtung (30) mit einem gummielastischen Ventilkörper in Form eines offenen becherförmigen Hohlzylinders (31) mit geschlitztem Bodenteil,
**dadurch gekennzeichnet**, daß
der Hohlzylinder (31) in der Kammer (11) axial beweglich ist, daß eine Feder (40) den Hohlzylinder (31) in Schließstellung mit seinem Öffnungsrand gegen das innere Ende einer Anschlußteil-Einstecköffnung (13) des Gehäuses (20) andrückt, deren Durchmesser größer als der Innenumfang des Öffnungsrandes des Hohlzylinders (31) ist und daß dabei der Bodenteil (34) in eine angepaßte zentrale Lochöffnung (56) eines in dem Gehäuse (20) fixierten Schließringes (50) hineingezogen ist, aus der der Bodenteil (34) zur Freigabe seiner durch Schlitzung (42) gebildeten Schenkel (43,44) gegen die Federkraft axial herausdrückbar ist.

2. Ansatzstück nach Anspruch 1,
**dadurch gekennzeichnet,** daß
der Hohlzylinder (31) mit kreisförmigem Querschnitt von einer Schraubenfeder (40) umgeben ist, deren eines Ende sich auf dem Schließring (50) abstützt und deren anderes Ende an dem Hohlzylinder (31) festgelegt ist.

3. Ansatzstück nach Anspruch 1 oder 2,
**dadurch gekennzeichnet,** daß
der Bodenteil (34) dick ausgebildet ist und die Schlitzung (42) des Bodenteiles (34) sich bis in die Mantelfläche des Hohlzylinders (34) erstreckt und daß das freie Ende des Bodenteiles (34) einen äußeren Ringkonus (38) aufweist, der in Schließstellung des Hohlzylinders (31) in eine konische Vertiefung (57) an dem patientenseitigen Ende der Lochöffnung (56) des Schließringes (50) im wesentlichen passend eingreift.

4. Ansatzstück nach einem der Ansprüche 1 bis 3,
**dadurch gekennzeichnet,** daß
ein kreiszylindrischer Längskanal (36) in dem Hohlzylinder (31) an der inneren Grundfläche des Bodenteiles (34) in einer symmetrischen Kegelvertiefung (37) endet und an seinem entgegengesetzten offenen Ende einen inneren Ringbund (41) aufweist.

5. Ansatzstück nach einem der Ansprüche 1 bis 4,
**dadurch gekennzeichnet,** daß
die Kammer (11) in dem Gehäuse (20) kreiszylindrisch ist und daß der Schließring (50) ein separates Teil aus zwei halbkreisförmigen Hälften bildet.

## Claims

1. A hub member for a rigid or a flexible medical conduit (21), consisting of a housing (20) with a chamber (11) open at both ends, one open end of said chamber being connected with said conduit (21), while the opposite open end thereof receives a connecting member, and consisting of a valve device (30) with a resilient valve body in the form of an open cup-shaped hollow cylinder (31) with a slit bottom portion,
characterized in that
said hollow cylinder (31) is axially displaceable in said chamber (11),
a spring (40) is provided that, in the closed position, presses the edge of the opening of said hollow cylinder (31) against the inner end of an insertion opening (13) for a connecting member, which is formed at said housing (20) and has a diameter larger than the inner circumference of the edge of the opening of said hollow cylinder (31), and
said bottom portion (34) extends into an adapted central hole (56) of a closing ring (50) fixed in said housing (20), through which hole said bottom portion (34) may be pushed axially against the spring action for releasing its legs (43, 44) formed by the slit (42).

2. The hub member of claim 1, characterized in that said hollow cylinder (31) of circular cross section is surrounded by a helical spring (40), one end of which is supported on said closing ring (50), while the other end is fixed at said hollow cylinder (31).

3. The hub member of claim 1 or 2, characterized in that said bottom portion (34) is thick and that said slit (42) of said bottom portion (34) extends into the lateral surface of said hollow cylinder (31) and that the free end of the bottom portion (34) has an outer annular cone (38) which, in the closed position of the hollow cylinder (31), engages substantially fittingly in a conical recess (57) at the proximal end of said hole (56) in said closing ring (50).

4. The hub member of one of claims 1 to 3, characterized in that a circular cylindrical longitudinal channel (36) in said hollow cylinder (31) ends in a symmetrical conical recess (37) in the basic surface of said bottom portion (34) and that it has an inner annular collar (41) at its opposite open end.

5. The hub member of one of claims 1 to 4, characterized in that said chamber (11) in said housing (20) is of circular cylindrical shape and that said closing ring (50) is a separate member of two semi-circular halves.

## Revendications

1. Embout, destiné à une conduite médicale rigide ou flexible (21), qui se compose d'un boitier (20), pourvu d'une chambre (11) ouverte à ses deux extrémités, dont une extrémité ouverte est reliée à la conduite (21) et dont l'extrémité ouverte opposée reçoit un élément de raccord, et d'un dispositif de soupape (21), pourvu d'un corps de soupape élastique en caoutchouc en forme de cylindre creux ouvert (31) à configuration de godet à une partie de fond fendue
caractérisé en ce que,
le cylindre creux (31) est mobile axialement dans la chambre (11), en ce qu'un ressort (40) pousse en position fermée le cylindre creux par son bord d'ouverture contre l'extrémité intérieure d'une ouverture d'insertion (13) et de raccord ménagée dans le boîtier (20), dont le diamètre est plus grand que la périphérie inférieure du bord d'ouverture du cylindre creux (31), et en ce que la partie de fond (34) est tirée vers l'intérieur dans un trou traversant central adapté (56) qui est ménagé dans une bague de fermeture (50) fixée dans le boîtier (20), hors duquel la partie de fond (34) peut être poussée vers l'extérieur axialement en opposition à la force de ressort en vue de libérer ses branches (43, 44) formées par une fente (42).

2. Embout selon la revendication 1,
caractérisé en ce que,
le cylindre creux (31) à section transversale circulaire est entouré par un ressort à boudin (40), dont une extrémité s'appuie sur la bague de fermeture (20) et dont l'autre extrémité est fixée au cylindre creux (31).

3. Embout selon la revendication 2,
caractérisé en ce que
la partie de fond (34) est d'une structure épaisse et en ce que la fente (42) de la partie de fond (34) s'étend jusque dans la surface d'enveloppe du cylindre creux (31) et en ce que l'extrémité libre de la partie de fond (34) comporte un cône circulaire extérieur (38) qui pénètre de façon sensiblement ajustée, dans une position de fermeture du cylindre creux (31), dans un creux conique (57) à l'extrémité coté patient du trou traversant (56) de la bague de fermeture (50).

4. Embout selon l'une des revendications 1 à 3,
caractérisé en ce que,
un canal longitudinal en cylindre circulaire (36) ménagé dans le cylindre creux (31) se termine, à la surface de base intérieure de la partie de fond (34), dans un creux conique symétrique (37), et comporte, à son extrémité ouverte opposée, un bourrelet annulaire intérieur (41).

5. Embout selon l'une des revendications 1 à 4,
caractérisé en ce que,
la forme de la chambre (11) dans le boîtier (20) est un cylindre circulaire et en ce que la bague de fermeture (50) constitue un élément séparé en deux moitiés en forme de demi-cercles.
